# EUROPEAN PATENT APPLICATION

(11) **EP 4 130 250 A1**
(43) Date of publication of application: **08.02.2023**
(21) Application number: 21780670.2
(22) Date of filing: 25.03.2021
(51) Int. Cl.: C12N 5/0775, C12N 5/0735, C12N 5/0789, C12N 5/10, C12Q 1/02

(54) **STABILIZER FOR STEM CELL CHROMOSOMES**

(30) Priority: 30.03.2020 JP 2020060490
(71) Applicant: Oriental Yeast Co., Ltd., Tokyo 174-8505 (JP)
(72) Inventor: GA Ayumi, Nagahama-shi, Shiga 526-0804 (JP); KINOSE Keita, Nagahama-shi, Shiga 526-0804 (JP); TOMIMORI Yoshiya, Nagahama-shi, Shiga 526-0804 (JP); YASUDA Hisataka, Nagahama-shi, Shiga 526-0804 (JP)
(74) Representative: Vossius & Partner Patentanwälte Rechtsanwälte mbB
(86) International application number: PCT/JP2021/012557
(87) International publication number: WO 2021/200545

(57) **Abstract**

The present invention provides a chromosome-stabilizing agent for stem cells containing a β-nicotinamide mononucleotide or a pharmaceutically acceptable salt thereof, or a solvate thereof, as an active ingredient. The present invention also provides a culture method for stem cells, including culturing stem cells in a culture medium containing a β-nicotinamide mononucleotide or a pharmaceutically acceptable salt thereof, or a solvate thereof.

## Description

### [Technical Field]

The present invention relates to a material capable of preventing the occurrence of chromosomal aberration in the process of culturing and passage of stem cells, and a chromosome-stabilizing agent for stem cells using the material.

### [Background Art]

Stem cells, typified by pluripotent stem cells, are undifferentiated cells having self-renewal ability and can be differentiated into various cells. In recent years, regenerative medicine, in which stem cells or cells induced to differentiate from stem cells are transplanted into damaged tissues of patients to regenerate the functions, has been actively studied. In regenerative medicine, it is necessary to prepare a large amount of stem cells and their differentiated cells, and thus the development of methods for efficiently proliferating stem cells and methods for efficiently differentiating stem cells has been actively attempted.

However, stem cells may develop chromosome abnormalities when cultured for a long period of time or when the number of passages is increased. Chromosome abnormalities include structural abnormalities such as translocations, inversions, partial duplications, partial deletions or the like, in addition to aneuploidy abnormalities such as monosomy in which two chromosomes are paired into one, trisomy in which two chromosomes are paired into three, or the like. When such chromosome abnormalities occur in stem cells, not only are the proliferation and differentiation capabilities held by the stem cells lost, but there is a risk of mutating into cancer cells or developing tumors when cells or tissues differentiated from the stem cells are used for regenerative medicine. Cancer cells and tumors sometimes have chromosome abnormalities, and in recent years, methods for detecting chromosome abnormality more accurately have been reported (see, for example, Patent Documents 1 and 2).

On the one hand, nicotinamide mononucleotide (NMN) is a biosynthetic intermediate metabolite of coenzyme NAD⁺. In recent years, it has been reported that NMN has the effect of improving insulin secretion in aging mice, the effect of dramatically improving insulin sensitivity and secretion in mouse models of type 2 diabetes caused by high-fat diet and aging (see, for example, Patent Document 3), and the effect of significantly enhancing the mitochondrial function of aged muscle, and the like. Furthermore, it has also been reported that culturing pluripotent stem cells in the presence of NMN can improve the proliferation ability and differentiation ability (see, for example, Patent Documents 4 and 5).

### [Citation List]

### [Patent Document]

[Patent Document 1] Japanese Unexamined Patent Application, First Publication No. 2019-213537
[Patent Document 2] Japanese Unexamined Patent Application, First Publication No. 2019-144097
[Patent Document 3] U.S. Pat. No. 7,737,158
[Patent Document 4] PCT International Publication No. WO 2018/143258
[Patent Document 5] PCT International Publication No. WO 2019/182044

### [Summary of Invention]

### [Technical Problem]

An objective of the present invention is to provide a material capable of preventing the occurrence of chromosome abnormalities in the process of culturing and passage of stem cells, a chromosome-stabilizing agent for stem cells, a culture method for stem cells and a chromosome-stabilizing method for stem cells, using the material.

### [Solution to Problem]

As a result of intensive research to solve the above problems, the present inventors found that the presence of a β-NMN improves the stability of chromosomes in stem cells and suppresses and prevents chromosome abnormalities, thereby completing the present invention.

That is, the present invention provides the following chromosome-stabilizing agent for stem cells, culture method for stem cells, and chromosome-stabilizing method for stem cells.
[1] A chromosome-stabilizing agent for stem cells, comprising a β-nicotinamide mononucleotide or a pharmaceutically acceptable salt thereof, or a solvate thereof, as an active ingredient.
[2] The chromosome-stabilizing agent according to [1], which is added to a stem cell culture medium in an amount of 0.01 to 5 mM in terms of β-nicotinamide mononucleotide.
[3] The chromosome stabilization agent according to [1] or [2], which is used for chromosome stabilization of one or more stem cells selected from the group consisting of an embryonic stem cell, an induced pluripotent stem cell, a mesenchymal stem cell and a hematopoietic stem cell.
[4] A culture method for stem cells, comprising culturing a pluripotent stem cell in a culture medium containing a β-nicotinamide mononucleotide or a pharmaceutically acceptable salt thereof, or a solvate thereof.
[5] The culture method according to [4], wherein a β-nicotinamide mononucleotide concentration of the culture medium is 0.01 to 5 mM.
[6] The culture method according to [4] or [5], wherein the stem cell is one or more selected from the group consisting of an embryonic stem cell, an induced pluripotent stem cell, a mesenchymal stem cell and a hematopoietic stem cell.
[7] A chromosome-stabilizing method for stem cells, comprising culturing stem cells in a culture medium containing a β-nicotinamide mononucleotide or a pharmaceutically acceptable salt thereof, or a solvate thereof.

### [Advantageous Effects of Invention]

The chromosome-stabilizing agent for stem cells according to the present invention can act on stem cells to improve the stability of the stem cell chromosomes and suppress or prevent the occurrence of chromosome abnormalities. Therefore, by including the chromosome-stabilizing agent in the culture medium, the stability of stem cell chromosomes can be improved, the occurrence of chromosome abnormalities can be suppressed or prevented, and the stem cells can be more stably cultured. In addition, by using the chromosome-stabilizing agent, it is possible to reduce the risk of canceration or tumorigenesis of cells and tissues differentiated from the stem cells.

### [Brief Description of Drawings]

FIG.1 (a) is a fluorescence photograph of cells stained with PI in Example 2, FIG.1 (b) is a fluorescence photograph of cells immunostained with anti-yH2A.X antibody in Example 2, and FIG.1 (c) is a merged photograph of (a) and (b).
FIG.2 is a graph showing the ratio of yH2A.X-positive cell nuclei in all PI-stained cells.

### [Description of Embodiments]

In the present invention and the present specification, stem cells refer to undifferentiated cells having self-renewal ability and differentiation ability (the ability to differentiate into various cell types), and examples thereof include pluripotent stem cells such as ES cells (embryonic stem cells), iPS cells (induced pluripotent stem cells) or the like, as well as somatic stem cells such as mesenchymal stem cells, hematopoietic stem cells, neural stem cells, skin stem cells or the like. Stem cells capable of differentiating into cells derived from ectoderm, mesoderm and endoderm, stem cells capable of differentiating into cells derived from mesoderm, and the like are preferable.

The chromosome-stabilizing agent for stem cells according to the present invention (hereinafter sometimes referred to as "chromosome-stabilizing agent according to the present invention") contains an NMN (chemical formula: C₁₁H_{1.5}N₂O₈ P) as an active ingredient, and when culturing and subculturing stem cells, it can be added to the culture medium. By culturing and subculturing stem cells in the presence of NMN, the stability of stem cell chromosomes can be improved, and the occurrence of chromosome abnormalities can be suppressed or prevented.

There are two optical isomers of NMN, α-type and β-type, and the NMN which is an active ingredient of the chromosome-stabilizing agent according to the present invention is a β-NMN (CAS number: 1094-61-7). The structure of β-NMN is shown below.

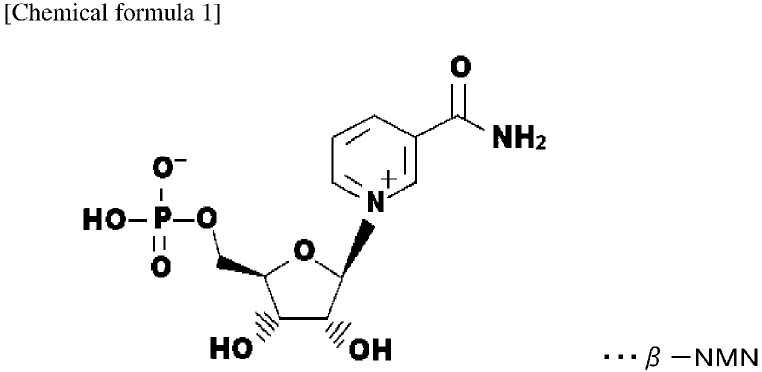

β-NMN as an active ingredient may be prepared by any method. For example, β-NMN artificially synthesized by a chemical synthesis method, an enzymatic method, a fermentation method or the like can be purified and used as an active ingredient. In addition, since β-NMN is a component widely present in living organisms, β-NMN obtained by extraction and purification from natural raw materials such as animals, plants, and microorganisms can also be used as an active ingredient. Further, commercially available purified β-NMN may also be used.

As the chemical synthesis method for synthesizing β-NMN, for example, β-NMN can be produced by reacting NAM with L-ribose tetraacetate, and phosphorylating the resulting nicotinamide mononucleotide. In addition, as the enzymatic method, for example, β-NMN can be produced from NAM and 5'-phosphoribosyl-1'-pyrophosphate (PRPP) by nicotinamide phosphoribosyltransferase (NAMPT). As the fermentation method, for example, β-NMN can be produced from NAM using the metabolic system of a microorganism expressing NAMPT.

The active ingredient of the chromosome-stabilizing agent according to the present invention may be a pharmaceutically acceptable salt of β-NMN. The pharmaceutically acceptable salt of β-NMN may be an inorganic acid salt or an organic acid salt having a basic site such as an amine. Examples of the acids constituting such acid salts include acetic acid, benzenesulfonic acid, benzoic acid, camphorsulfonic acid, citric acid, ethenesulfonic acid, fumaric acid, gluconic acid, glutamic acid, hydrobromic acid, hydrochloric acid, isethionic acid acids, lactic acid, maleic acid, malic acid, mandelic acid, methanesulfonic acid, mucic acid, nitric acid, pamoic acid, pantothenic acid, phosphoric acid, succinic acid, sulfuric acid, tartaric acid, p-toluenesulfonic acid, and the like. In addition, the pharmaceutically acceptable salt of β-NMN may be an alkali salt or an organic salt having an acidic site such as a carboxylic acid. Examples of bases constituting such acid salts include bases which are alkali metal salts or alkaline earth metal salts and which are induced from bases such as sodium hydride, potassium hydroxide, calcium hydroxide, aluminum hydroxide, lithium hydroxide, magnesium hydroxide, zinc hydroxide, ammonia, trimethyl ammonia, triethyl ammonia, ethylene diamine, lysine, arginine, ornithine, choline, N,N'-dibenzyl ethylenediamine, chloroprocaine, procaine, diethanolamine, N-benzylphenethylamine, diethylamine, piperazine, tris(hydroxymethyl)-aminomethane, and tetramethyl ammonium hydroxide.

The active ingredient of the chromosome-stabilizing agent according to the present invention may be a solvate of free β-NMN or pharmaceutically acceptable salts thereof. Examples of solvents that form the above-mentioned solvate include water, ethanol, and the like.

The chromosome-stabilizing agent according to the present invention may contain other active ingredients in addition to β-NMN. As for other active ingredients used in combination with β-NMN, only one kind may be used, or two or more kinds may be used in combination. Such other active ingredients may be appropriately selected from the ingredients known to increase stem cell survival rate and proliferation efficiency, such as albumin, ascorbic acid, α-tocopherol, insulin, transferrin, sodium selenite, ethanolamine, Rock inhibitors or the like; ingredients known to increase the efficiency of stem cell differentiation, such as valproic acid, dimethylsulfoxide, dexamethasone, butyric acid, trichostatin A, GSK3 inhibitors, BMP inhibitors, Wnt inhibitors, activin, noggin or the like, and the like, and used.

By incorporating the chromosome-stabilizing agent according to the present invention in a culture medium when culturing stem cells, it is possible to proliferate the stem cells while improving the stability of stem cell chromosomes and suppressing or preventing the occurrence of chromosome abnormalities. The amount of the chromosome-stabilizing agent according to the present invention contained in the culture medium for stem cells is not particularly limited and can be appropriately adjusted in consideration of the type of pluripotent stem cells, the balance with other components of the culture medium and the like, as long as it is an amount that provides a sufficient concentration for suppressing the number of stem cells with chromosome abnormalities as compared to the case of culturing in a culture medium that does not contain the chromosome-stabilizing agent. In a case where the concentration of β-NMN in the culture medium is too low, the chromosome-stabilizing effect may be weak, and in a case where β-NMN is excessively contained, the growth of cells may be suppressed conversely. The content of the chromosome-stabilizing agent according to the present invention in a culture medium is preferably 0.01 to 5 mM, more preferably 0.05 to 2 mM, even more preferably 0.1 to 1 mM, in terms of β-NMN concentration. When the β-NMN concentration is within the above range, the stem cell chromosome can be sufficiently stabilized.

Culturing of stem cells in the presence of the chromosome-stabilizing agent according to the present invention can be performed by general methods except that the culture medium contains the chromosome-stabilizing agent according to the present invention. For example, as the culture medium, it is possible to use a medium generally used for maintenance or growth of stem cells, and a medium used for culture of animal cells. In addition, it is possible to use various commercially available culture media for stem cells. In the present invention, examples of the media that contain the chromosome-stabilizing agent according to the present invention and are used for culture of stem cells include Eagle's Minimal Essential Medium (MEM), Dulbecco's modified Eagle's medium (DMEM), Minimum Essential Medium Eagle-α (αMEM), Iscove's Modified Dulbecco's Medium (IMDM), F-12 medium, F-10 medium, DMEM/F12 medium, RPMI-1640 medium, mesenchymal cell basal medium (MSCBM), E8 (Essential 8) medium, TeSR-E8 medium, mTeSR1 medium, MCDB medium and the like. If necessary, amino acids, inorganic salts, vitamins, antibiotics, and the like may be added to these media.

In these culture media, a component known to enhance survival efficiency or growth efficiency of stem cells, or a component known to have an effect of maintaining an undifferentiated state of stem cells, and the like may be appropriately contained in addition to the chromosome-stabilizing agent according to the present invention. As these components, components described above can be used.

In addition, culture conditions can be set as general culture conditions for culturing animal cells, and may be suitably modified as necessary. For example, culture can be performed at a culture temperature of 30 to 40°C, a CO₂ concentration of 1 to 10% by volume, and an O₂ concentration of 0.1 to 25% by volume.

The stem cells whose chromosomes can be stabilized by the chromosome-stabilizing agent according the present invention are preferably animal-derived stem cells, more preferably mammal-derived stem cells, and even more preferably human-derived stem cells. The stem cells whose chromosomes can be stabilized by the chromosome-stabilizing agent according to the present invention are preferably animal-derived ES cells, iPS cells or mesenchymal stem cells, more preferably mammals-derived ES cells, iPS cells or mesenchymal stem cells, and even more preferably human-derived ES cells, iPS cells or mesenchymal stem cells.

### [EXAMPLES]

Next, the present invention will be described in more detail with reference to examples, but the present invention is not limited to the following examples.

### [Example 1]

The effect of β-NMN on chromosomes was investigated during the culture of mesenchymal stem cells. As the mesenchymal stem cells, human adipose tissue-derived mesenchymal stem cells (ADSC) (product number: PT-5006, manufactured by Lonza) from two donors were used.

In the culture of mesenchymal stem cells, a medium obtained by mixing DMEM medium and MCDB medium at 1:1 was used as a basal medium (see Japanese Patent No. 5804385), and a medium obtained by adding FGF, PDGF, TGF-β, HGF, EGF, phospholipids, fatty acids, etc. to the basal medium was used as a base medium. The β-NMN-supplemented medium was prepared by adding 0.25 mM of β-NMN to the base medium.

### <Subculture>

Mesenchymal stem cells were seeded at 5×10³ cells/cm² on a culture dish previously coated with 2.5 µg/cm² fibronectin, and cultured using the basal medium. One day after seeding, the medium was changed to the base medium or the β-NMN-supplemented medium. Thereafter, the medium was changed once every 2 or 3 days. Subculture was performed when the cell density in the culture dish reached a confluency of about 80% to 90%.

First, after removing the culture supernatant and washing with 1×PBS (-), the cells were detached using 1×Tryple select (product number: 12563011, manufactured by Thermo Fisher) and placed for 4 minutes under conditions of 37°C and 5% CO₂. After the cells were singled by pipetting, the base medium or the β-NMN-supplemented medium was added, and centrifugation was performed. After suspending the recovered cells in each medium, the cells were counted and seeded on a pre-coated culture dish at 5×10³ cells/cm². Medium exchange was performed once every 2 or 3 days using the base medium or the β-NMN-supplemented medium.

After performing the above subculture 5 times, the cells were frozen using the cell cryopreservation solution "STEM-CELLBANKER DMSO Free GMP grade" (product number: CB061, manufactured by Nippon Zenyaku Kogyo Co., Ltd.), according to the manufacturer's recommended protocol.

The frozen cells were seeded in the base medium or the β-NMN-supplemented medium in a coated culture dish at 5×10³ cells/cm², and the medium was changed 1 day after seeding. After that, the medium was changed once every 2 or 3 days.

The cells were subcultured once and seeded in a T25 flask at 5×10³ cells/cm². Three days after seeding, the T25 flask filled with the medium was sent to a chromosome safety test consignment institution (Japan Gene Research Institute, Inc.) for chromosome analysis.

### <Chromosome stability test>

The chromosomal state of the cultured mesenchyme stem cells was confirmed by the G-band staining method. Table 1 shows the measurement results of the number of chromosomes, and Table 2 shows the results of karyotype analysis. In addition, the analysis results are described according to the chromosome karyotype description method (ISCN: International System for Human Cytogenomic Nomenclature) based on international regulations.

**[Table 1]**

| | β-NMN | Number of chromosomes | Number of cells |
|---|---|---|---|
| Test group 1 | - | 46 | 46 |
| | | 47 | 4 |
| | | | 50 in total |
| Test group 2 | - | 45 | 1 |
| | | 46 | 49 |
| | | | 50 in total |
| Test group 3 | + | 46 | 50 |
| | | | 50 in total |
| Test group 4 | + | 46 | 50 |
| | | | 50 in total |

**[Table 2]**

| | β-NMN | Number of chromosomes | Karyotype analysis results | Number of cells |
|---|---|---|---|---|
| Test group 1 | - | 46 | 46, XY | 17 |
| | | 47 | 47, XY, +5 | 3 |
| | | | | 20 in total |
| Test group 2 | - | 45 | 45, XX, -7, -18, +mar | 1 |
| | | 46 | 46, XX | 19 |
| | | | | 20 in total |
| Test group 3 | + | 46 | 46, XY | 20 |
| | | | | 20 in total |
| Test group 4 | + | 46 | 46, XX | 20 |
| | | | | 20 in total |

Test group 1 shows the mesenchymal stem cells cultured in the base medium (ADSC donor 1, male), and Test group 2 shows the mesenchymal stem cells cultured in the base medium (ADSC donor 2, female). On the other hand, Test group 3 shows the mesenchymal stem cells cultured in the β-NMN-supplemented medium (ADSC donor 1, male), and Test group 4 shows the mesenchymal stem cells cultured in the β-NMN-supplemented medium (ADSC donor 2, female).

In the mesenchymal stem cells cultured in the base medium, abnormal numbers of chromosomes were observed in both donors (Test groups 1 and 2) (Table 1). That is, as a result of measuring the number of chromosomes of 50 cells in each test group, in Test group 1, the number of chromosomes increased by one from 46 to 47 in 4 cells. Further, in Test group 2, the number of chromosomes decreased by one from 46 to 45 in one cell. On the other hand, the mesenchymal stem cells cultured in the β-NMN-supplemented medium had 46 chromosomes in all cells of both donors (Test groups 3 and 4), which is the normal number of human chromosomes. As a result of karyotype analysis, the number of abnormal chromosomes was different in different donors (Table 2). As a result of karyotype analysis of 20 chromosomes in each test group, in Test group 1, three cells had one extra chromosome No. 5, resulting in trisomy. In Test group 2, in one cell, chromosomes No. 7 and No. 18 decreased by one each, and a marker chromosome of unknown origin increased by one. In contrast, in the mesenchymal stem cells cultured in the β-NMN-supplemented medium, no abnormality was observed in the karyotype of the chromosomes of the two donors (Test groups 3 and 4) (Table 2). In the case of normal, it was described as "46, XY" for the male donor in Test group 3, and described as "46, XX" for the female donor in Test group 4. These results confirmed that β-NMN has the effect of improving the stability of stem cell chromosomes.

### [Example 2]

The effect of β-NMN on chromosomes was investigated during culture of human pluripotent stem cells (iPSCs).

### <Culture medium/culture method>

201B7 strain was used as human pluripotent stem cells (iPSCs), and cultured in a 24-well cell culture plate pre-coated with an extracellular matrix (Matrigel: Corning) under conditions of 5% CO₂ and 37°C until the cell density reached about 80%.

As the medium, a base medium obtained by adding cytokines and salts (insulin, transferrin, TGFβ, FGF, sodium selenite, ascorbic acid, bicarbonate, etc.) that are known to be necessary for the maintenance subculture of iPSCs, to the basal medium (DMEM-F12) was used. A β-NMN-free group (NMN (-)) in which the base medium was used, and a β-NMN-supplemented group (NMN (+)) in which β-NMN was added to the base medium to a final concentration of 1 mM, were used.

NCX4016 (Sigma-Aldrich, Product No. SML1669) was added to each of the β-NMN-free and β-NMN-supplemented groups to a final concentration of 5 µM, and incubated for 2 hours to induce chromosomal damage. Subsequently, for the purpose of investigating the degree of chromosomal damage, cells with damaged nuclear genomic DNA were detected by phosphorylated histone H2AX (yH2A.X) labeling.

### <Immunostaining>

Cells incubated with NCX4016 were fixed with 0.99% formaldehyde, permeabilized with 0.10% Triton X-100, followed by immunostaining using an anti-yH2A.X antibody (manufactured by Thermo Fisher, Product No. 14-9865-82) and an anti-mouse IgG antibody fluorescently labeled with FITC (manufactured by Thermo Fisher, Product No. 11-4015-82) to detect yH2A.X by observing with a fluorescence microscope. Further, the DNA was fluorescently stained with propidium iodide (manufactured by Nacalai Tesque, product number 19174-31) so that the nuclei of all cells could be observed at the same time.

### <Results>

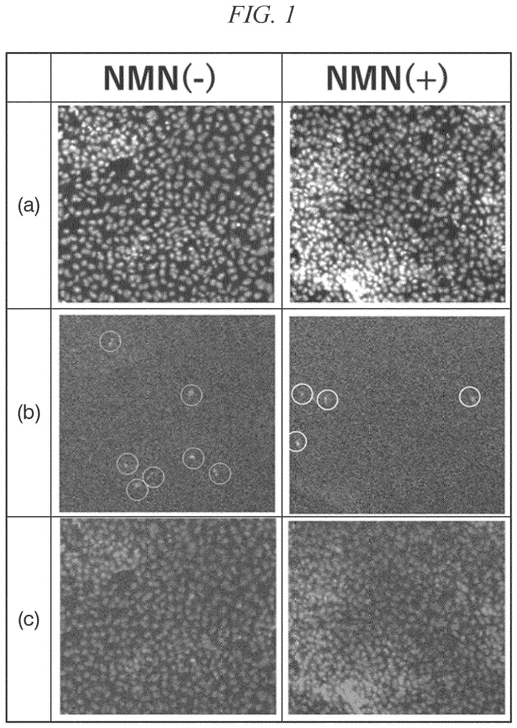

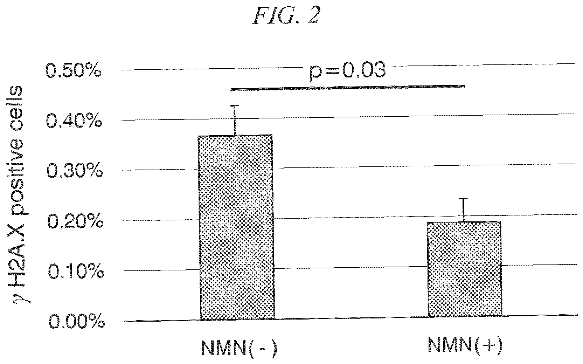

As shown in FIG.1 (b), yH2A.X-stained cell nuclei (indicated by circles) were significantly smaller in the β-NMN-supplemented group (NMN (+)) than in the β-NMN-free group (NMN (-)). Further, as shown in FIG.2, the ratio of yH2A.X-positive cell nuclei was statistically significantly smaller in the β-NMN-supplemented group (NMN (+)) than in the β-NMN-free group (NMN (-)) (hypothesis testing for the difference in the population proportions, p <0.05, n>8000). In FIG.2, the bar graph shows the ratio of yH2A. X-positive cell nuclei in each group, and the error bars represent the width of the standard error. This result indicates that the addition of β-NMN reduces the damage to genomic DNA that causes chromosome abnormalities in stem cells.

## Claims

1. A chromosome-stabilizing agent for stem cells, comprising a β-nicotinamide mononucleotide or a pharmaceutically acceptable salt thereof, or a solvate thereof, as an active ingredient.

2. The chromosome-stabilizing agent according to claim 1, which is added to a stem cell culture medium in an amount of 0.01 to 5 mM in terms of β-nicotinamide mononucleotide.

3. The chromosome stabilization agent according to claim 1 or 2, which is used for chromosome stabilization of one or more stem cells selected from the group consisting of an embryonic stem cell, an induced pluripotent stem cell, a mesenchymal stem cell and a hematopoietic stem cell.

4. A culture method for stem cells, comprising culturing a pluripotent stem cell in a culture medium containing a β-nicotinamide mononucleotide or a pharmaceutically acceptable salt thereof, or a solvate thereof.

5. The culture method according to claim 4, wherein a β-nicotinamide mononucleotide concentration of the culture medium is 0.01 to 5 mM.

6. The culture method according to claim 4 or 5, wherein the stem cell is one or more selected from the group consisting of an embryonic stem cell, an induced pluripotent stem cell, a mesenchymal stem cell and a hematopoietic stem cell.

7. A chromosome-stabilizing method for stem cells, comprising culturing stem cells in a culture medium containing a β-nicotinamide mononucleotide or a pharmaceutically acceptable salt thereof, or a solvate thereof.
